# EUROPEAN PATENT APPLICATION

(11) **EP 2 433 649 A2**
(43) Date of publication of application: **28.03.2012**
(21) Application number: 11189431.7
(22) Date of filing: 11.09.2007
(51) Int. Cl.: A61K 39/395, A61P 29/00, C07K 16/28

(54) **Treatment of multiple sclerosis (MS) with campath-1H**

(30) Priority: 13.09.2006 US 844251 P; 14.09.2006 EP 06090169
(62) Divisional of application: 07802348.8
(71) Applicant: Alcafleu Management GmbH & Co. KG, 12529 Schönefeld OT Waltersdorf (DE); Genzyme Corporation, Cambridge, MA 02142 (US)
(72) Inventor: Sachse, Andreas, 10709 Berlin (DE); Margolin, David Harris, Somerville, MA 02144 (US)
(74) Representative: Adams, Harvey Vaughan John

(57) **Abstract**

A method for treatment of multiple sclerosis (MS) with Campath-1H with significant efficacy and a favourable safety profile is which offers an acceptable benefit/risk ratio. Especially described is the use of Campath-1H (alemtuzumab) for the production of a medicament for the treatment of multiple sclerosis (MS), comprising a first treatment cycle followed by at least one further treatment cycle of Campath-1 (alemtuzumab), in which each treatment cycle comprises 1-5 daily doses which are applied on consecutive days, wherein the daily dose is >0 and ≤ 12 mg, and wherein each treatment cycle is separated from the next cycle by at least 1 - 24 months. Also are treatment regimens comprising the administration of less than 12 mg/day of Campath-1 for a period of 1-5 consecutive days.

## Description

The present invention relates to a method for the treatment of multiple sclerosis (MS) with Campath-1H, with significant efficacy and a favourable safety profile which offers an acceptable benefit/risk ratio. It also relates to the use of Campath-1 H for the production of a medicament for the treatment of multiple sclerosis (MS).

Multiple sclerosis (MS) is an inflammatory demyelinating disease of the central nervous system that affects as many as 2.5 million people worldwide. The pathogenesis of MS remains poorly understood but is believed to arise from the interplay of polygenic inherited susceptibility and an unidentified environmental agent(s). It is approximately twice as common among women as men. Worldwide, its prevalence varies geographically and, within the same country, between different racial groups. Prevalence is highest amongst Caucasians in countries distant from the Equator, for instance Scotland and Scandinavia. Peak incidence is within the third and fourth decades; it is extremely uncommon to make a new diagnosis in patients over the age of 60 years [National Multiple Sclerosis Society, General Information, Just the Facts: 2000-2001. National Multiple Sclerosis Society; 2001.].

Campath-1H (alemtuzumab) is a recombinant DNA-derived humanized monoclonal antibody that is directed against the 21-28 kD cell surface glycoprotein, CD52. CD52 is an abundant molecule (approximately 5 × 10⁵ antibody binding sites per cell) present on at least 95% of all human peripheral blood lymphocytes and monocyteslmacrophages [Hale G. et al., The CAMPATH-1 antigen (CD52). Tissue Antigens 1990;35:118-127].

Campath-1 H is disclosed in US patent US 5,846,534, wherein a humanized antibody which binds effectively to the antigen CD52 as well as a method of treating a human patient having a lymphoid malignancy with such an antibody is described. Procedures for preparation and testing of such an antibody are disclosed.

Campath-1H (alemtuzumab, Campath® or MabCampath®) is approved for the treatment of B-cell chronic lymphocytic leukaemia (B-CLL) in patients who have been treated with alkylating agents and who have failed fludarabine therapy. As labelled for treatment of CLL, Campath therapy is initiated at a dose of 3 mg administered as a 2 hour i.v. infusion daily. When the Campath 3 mg daily dose is tolerated, the daily dose is escalated to 10 mg and continued until tolerated. When the 10 mg dose is tolerated, the maintenance dose of Campath 30 mg/day is administered three times per week on alternate days (e.g., Monday, Wednesday, and Friday) for up to 12 weeks (see Campath® package insert).

Clinical studies have shown that the Campath-1H antibodies are also active in a variety of other diseases including graft-versus-host disease, organ transplant rejection, rheumatoid arthritis, and other autoimmune diseases, as well as in non-Hodgkin's lymphoma and leukemias [Hale G, Vllaldmann H. From laboratory to clinic: The story of Campath-1 H in antibodies in the clinic. In: George AJT, Ureli C, ed. Methods in Molecular Medicine. Diagnostic and Therapeutic Antibodies. NJ: Humana Press; 2000;40:319-323].

Hale and Waldmann were the first to disclose the use of Campath-1 H in multiple sclerosis. In US patent 6,120,766, Hale and Waldmann claim a method for the treatment of multiple sclerosis in a human subject which comprises administering an effective amount of Campath-1 H and an effective amount of a steroid (e.g. hydrocortisone or methylprednisolone). In that patent, they describe a 43 year old female with chronic progressive MS who had been treated with high doses of i.v. methylprednisolone (second course: 500 mg per day over 5 days) with limited improvement. The patient received 10 doses of Campath-1 H over 12 days (2 mg/day for five days, 2 days rest, then 10 mg/day for five days). Fever and headache were reported as adverse events during administration of the first 2 mg and 10 mg doses. One and two months after Campath-1 H administration the Kurtzke neurological status of the patient had improved and the improvement was maintained 18 months after the treatment.

Since then, Campath-1 H has been used in a variety of clinical studies in patients with primary progressive MS and secondary progressive MS (PPMS and SPMS, respectively). For example, in 1994, T. Moreau et al. reported the treatment of six SPMS patients and 1 PPMS patient with Campath 1 H at 12 mg/day for 1 a days *(*Lancet (1994), 344:298-301).

In 1996, T. Moreau et al. described the treatment of twelve SPMS and one PPMS patient with Campath-1 H using doses of 2 mg/day for 5 days and then 10 mg/day for 5 days, or using 12 mg day for 10 days, or using 20 mg/day for 5 days *(*Brain (1996),119:225-237). They reported that serum cytokine release, coinciding with the first infusion of Campath-1 H and the induction of lymphopaenia, is associated with transient symptomatic deterioration and altered conduction through previously affected CNS pathways.

In 1999, Coles et al. reported the treatment of 29 patients with SPMS using a dose of 20 mg/day for 5 days (Ann. Neurol. (1999), 46:296-304). They observed a transient rehearsal of previous or current symptoms during the first dose of Campath-1 H. About half the patients experienced progressive disability and increasing brain atrophy, attributable on the basis of MRI spectroscopy to axonal degeneration. Later in 1999, Coles et al. reported on the long term follow-up of 27 of the patients reported in a previous study (Lancet (1999), 354:1691-95). One third of the patients had developed antibodies against the thyrotropin receptor and carbimazole-responsive autoimmune hyperthyroidism (i.e., Graves' disease).

In 2003, Coles et al. reported that of the 36 SPMS patients who had received Campath 1 H since 1991, their relapse rate remained suppressed during a mean of 7 years of follow-up but their disability had continued to progress. In addition, one third (1/3) of the patients had developed Graves' disease (Neurology 60 March 2003 (Suppl. 1). They also reported the treatment of 22 patients with relapsing remitting MS (RRMS). A later report of these 22 RRMS patients confirmed that they had received a dose of 20mg/day for 5 days and elective re-treatment was offered after 12-18 months at 20 mg/day for 3 days (Clinical Neurology and Neurosurgery (2004), 106:270-274). The principal adverse event was Graves' disease which developed within 5-21 months of the first treatment (94 patients) and two years after the second cycle (1 patient) in a total of 15 of the 57 patients (27%)(one patient had Grave's disease before receiving Campath-1H).

In 2004, O'Donnell et al. reported at the Art and Science of MS Meeting held in Toronto on an ongoing trial comparing two dose levels of Campath-1 H to interferon beta-1a (Rebif®, Ares-Serono) in patients with early active RRMS. In this trial, (CAMMS223), Campath-1 H was administered at a dose of 12 mg/day (low dose) or 24 mg/day (high dose) for five days. The interferon beta-1 a patients received three s.c. injections per week as indicated in the product label (Rebif®).

Interim results from the CAMMS223 trial were announced by Genzyme Corporation and Schering AG Germany on September 16, 2005. These results were derived from a pre-specified efficacy and safety interim analysis conducted after one year of treatment for all patients in the planned three year trial. Patients were treated with Campath-1 H at low (12 mg/day) or high (24 mg/day) doses administered over five days in once a year intravenous infusion regimens, or interferon beta-1a administered three times per week as indicated in its product label. At 12 months, patients on Campath-1 H received a dose of 12 or 24 mg/day for three days. Patients taking Campath in both the high and low doses experienced at least a 75% reduction in the risk for relapse after at least one year of follow up when compared to patients treated with interferon beta-1a. The Campath patients additionally experienced at least a 60% reduction in the risk for progression of clinical significant disability compared to Rebifi®. However, three cases of severe idiopathic thrombocytopenic purpura (ITP) were reported (two in the high dose group and one in the low dose group).

Also in 2005, Fox et al. reported during ECTRIMS on a study of high-dose Campath in 45 patients with active RRMS who had failed licensed IFN-beta therapies. Campath was given at 24 mg/day for 5 days and then repeated after one year at 24 mg/day for 3 days. One drug-related serious adverse event occurred (neutropenia and pneumonia) and abnormal thyroid values were found in several patients.

In summary, administration of Campath-1 H at to patients with MS has shown efficacy in treating the disease, but such administration has also been associated with adverse events, which may include infectious and auto-immune complications. Thus, there remains a need for Campath-1 H regimens which result in significant efficacy in this patient population (i.e. reduction in risk for relapse and/or reduction in risk for progression of clinical significant disability) while having an acceptable safety profile.

### SUMMARY OF THE INVENTION

The invention relates to a method for the treatment of multiple sclerosis (MS) in a patient, comprising administration of a first cycle of Campath-1H followed by at least one further cycle of Campath-1 H, in which each treatment cycle comprises 1-5 doses which are applied on consecutive days, wherein the daily dose is >0 and ≤ 12 mg, and wherein each treatment cycle is separated from the next cycle by at least 1-24 months.

In some embodiments, patients are re-treated on a fixed time course, e.g., at 6, 12, 18 or 24 months after the first treatment. In other embodiments, patients are re-treated only once evidence of renewed MS activity has been observed.

In some embodiments, re-treatment occurs at the same dose and duration as the initial dose. In other embodiments, re-treatment occurs at the same dose for a different duration, or at a different dose for the same duration, as the initial cycle of treatment.

The invention also relates to a method for the treatment of multiple sclerosis in a patient, comprising administration of Campath-1H at a dose of less than 12 mg/day for a period of 1-5 days.

The invention also relates to the use of Campath-1 H for the production of a medicament to be administered according to the methods described herein.

The methods and uses of this invention are applicable to patients with relapsing MS as well as patients with progressive MS.

The foregoing summary and the following detailed description are exemplary and explanatory only and are not restrictive of the invention as claimed.

### BRIEF DESCRIPTION OF THE FIGURES

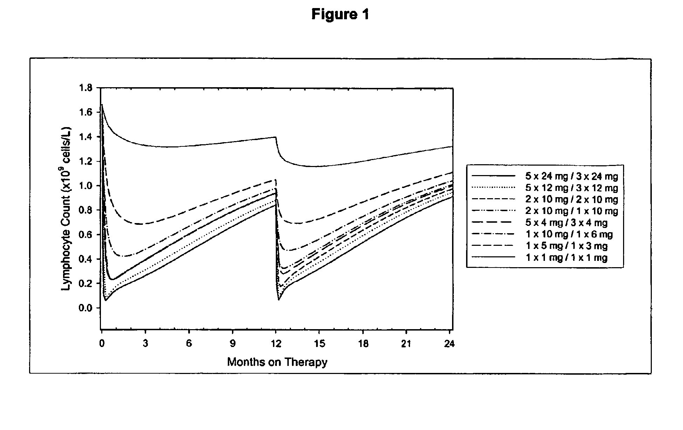

- Figure 1: presents a line plot of the simulated dosing regimens over a 24 month period. In the legend, the first set of numbers indicate the number of days by the daily dose. For example, 5 × 12 mg is 5 days of 12 mg infused over a 4 hour period. The second set of numbers are the retreatment doses on Month 12.

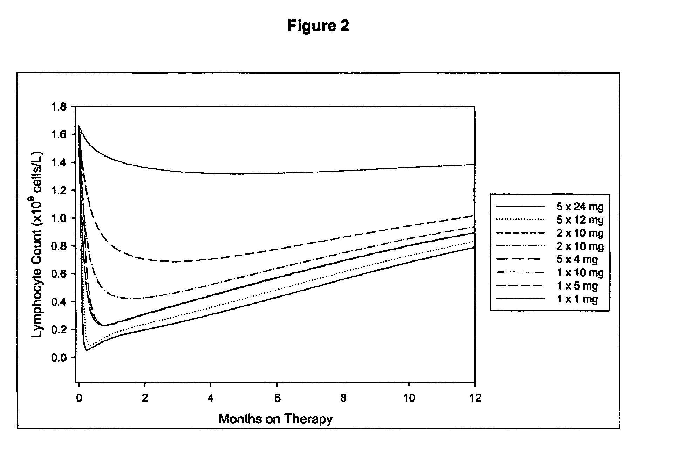

- Figure 2: presents a line plot of the simulated dosing regimens over a 12 month period.

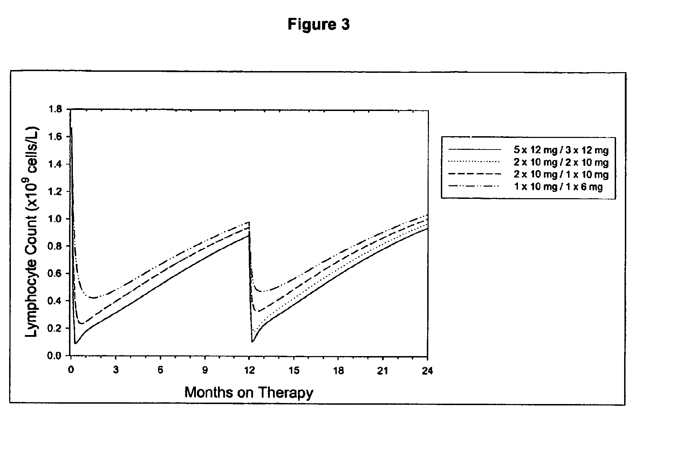

- Figure 3: presents a line plot of selected dosing regimens from Figure 1. Data for the two 10 mg doses administered during the first cycle are overlaid over each other and not discernible from each other.

### DETAILED DESCRIPTION OF THE INVENTION

As used herein, the term "Campath-1H" refers to the monoclonal antibody of the same name disclosed in US patent 5,846,534 (also known as alemtuzumab) as well as human or humanized monoclonal antibodies having the same CDR sequences as Campath-1 H.

In one aspect, the invention relates to the use of Campath-1 H for the production of a medicament for the treatment of relapsing MS patients, characterised in that the Campath-1 H is administered at a dose of less than 12 mg/day for a period of 1-5 consecutive days.

In another aspect, the invention relates to a method for treating relapsing MS patients which comprises the administration of Campath-1H at a dose of less than 12 mg/day for period of 1-5 consecutive days.

In another aspect, the invention relates to a method for treating MS patients which comprises the administration of Campath-1 H at a dose of less than 12 mg/day for a period of 1-5 consecutive days and then re-treating such patients using a treatment regimen equal to or less than the original regimen in either dose (mg/day) and/or duration (number of days).

In certain embodiments, the Campath-1H is administered at a dose of 11, 10, 9, 8, 7, 6, 5 or 4 mg/day for a period of 5 days. In other embodiments, the Campath is administered at a dose of 11, 10, 9, 8, 7, 6, 5 or 4 mg/day for periods of 2 or 3 or 4 days.

In another aspect, the invention relates to a method for treatment of MS, which comprises the cyclic application of Campath-1 H) in daily doses of up to 12 mg/day over a period of 1-5 days with each treatment cycle being separated from the prior cycle (i.e. Campath-1 H dosing) by at least one month. In certain embodiments, the Campath-1 H is administered at a dose of 2 - 10 mg per day.

In various embodiments, daily doses can remain the same for each cycle of treatment or may differ for the different treatment cycles (e.g. 10 mg/d for first cycle, 5 mg/d for subsequent cycles). Also contemplated by the inventors are daily doses that vary within one treatment cycle e.g., by escalation (e.g. 8 mg on first day, 10 mg on second day and 12 mg on third day and so on), or vice-versa.

The number of consecutive days of treatment (i.e. dosing) per cycle is normally 1-5. In certain embodiments, a cycle is 1-3 days. The number of dosing days can remain the same for each cycle or may differ for the different treatment cycles (e.g. 3 days for first cycle, 2 days for subsequent cycles). Less dosing days per cycle is expected to result in improved patient convenience/ acceptance and reduced treatment cost.

In another aspect, the invention relates to the use of Campath-1 H for the production of a medicament for the treatment of MS, which comprises the cyclic application of Campath-1 H) in daily doses of up to 12 mg/day over a period of 1-5 days with each treatment cycle being separated from the prior cycle (i.e. Campath-1 H dosing) by at least 1 month.

In some embodiments directed to cyclic application, the consecutive treatment cycles are separated by at least 3 or 6 months. In other embodiments, they are separated by at least 18 or 24 months. In certain embodiments, the number of consecutive treatment cycles is not limited so that lifelong treatment is potentially possible. In other embodiments, the number of treatment cycles is limited to 2 - 10 or 2 - 5 cycles.

In some embodiments, re-treatment occurs on a fixed time course, e.g., at 6, 12, 18 or 24 months after initial treatment.

In another aspect of the invention, re-treatment (i.e. application of an additional treatment cycle of 1-5 consecutive daily doses of ≤ 12 mg) only occurs once evidence of renewed MS activity in the respective patient is observed. This treatment regimen is referred to herein as "re-treatment as needed."

Evidence of renewed MS activity may be determined based on the professional judgement of the treating clinician, using any means that may be available to such clinician.

A variety of techniques are currently available to clinicians to diagnose renewed MS activity including, without limitation, by clinical means (relapse or progression of neurological disability) or by magnetic resonance imaging (MRI) of the brain or spinal cord. As is well understood by medical practitioners, disease activity detected via MRI may be indicated by the occurrence of new cerebral or spinal lesions on T1 (enhanced or non-enhanced)- or T2 weighted images or by the increase of the volume of such lesions. As diagnostic methods for MS are continually evolving, it is anticipated there may be additional methods in the future that will detect renewed MS activity (e.g. magnetization transfer ratio or MR-spectroscopy). The particular diagnostic method used to detect renewed MS activity is not a limitation of the claimed invention.

In certain embodiments, repeated MRIs are performed in fixed intervals after a treatment cycle in order to determine whether re-treatment of any given patient is necessary and the optimal time point for re-treatment of such patient. In general, it is desirable for re-treatment to occur before the disease re-manifests clinically.

This "re-treatment as needed" strategy is expected to maximize the benefit/risk ratio of the treatment regimens disclosed herein by potentially avoiding unnecessary drug exposure in patients with sustained MS suppression.

In embodiments which include re-treatment, it may be immediately initiated prior to or subsequent to cessation of any symptomatic treatment (e.g. steroids) which may have been administered for the acute relapse (i.e. no fixed interval (time period) between subsequent treatment cycles).

In certain embodiments, re-treatment upon renewed MS activity is only performed if at least 3-24 months have passed since the last treatment cycle.

In another aspect, the invention relates to the use of Campath-1 H for the production of a medicament for the treatment of MS, which comprises the cyclic application of Campath-1 H in daily doses of up to 12 mg/day over a period of 9-5 days, wherein re-treatment only occurs once evidence of renewed MS activity in the respective patient is observed.

All of the methods and uses of the invention are applicable to both relapsing as well as progressive forms of multiple sclerosis (MS). Patients with relapsing forms of MS are currently expected to respond more favourably than those with progressive forms.

MS patients amenable to treatment may be patients who were originally treated with other drug(s) or patients who have not received prior MS therapy (i.e. treatment (drug) naïve patients).

In the methods and uses of the invention, Campath-1 H may be administered via any acceptable route including, without limitation, via parenteral administration (e.g. intravenous, subcutaneous, intramuscular, intraperitoneal, nasal, pulmonary). In certain embodiments, Campath-1 H is administered intravenously (i.v.) or used for the production of a medicament to be administered intravenously.

In cases where premedication is desired, any drugs known to those skilled in the art to be effective for such purpose, such as for example steroids (e.g. methylprednisolone), acetaminophen and antihistamines (e.g. diphenhydramine) may be applied before, during or after infusion to manage infusion related side effects. In certain embodiments, only low doses of such drugs are utilized during the first 1-3 days of application during each treatment cycle. In other embodiments, no concomitant medication is administered.

Campath-1H treatment according to the methods of the invention, and uses of Campath-1 H for the production of medicaments according to the invention, potentially lead to a low rate of (serious) adverse events. Consequently MS treatment regimens according to the method of the invention are expected to result in an acceptable benefit/risk ratio.

According to the instant invention, Campath-1H is administered, or used for the production of a medicament to be administered, in a suitable pharmaceutical formulation containing appropriate excipients as known to those skilled in the art. The current Campath® (MabCampath®) formulation represents one example of such a suitable product (see Campath® package insert). In addition to a solution, Campath-1 H may also be formulated as a freeze-dried product which is reconstituted prior to use.

The formulation is preferably provided in vials or plastic bags (mainly for i.v. use) but other standard containers as known to those skilled in the art can also be used depending on the route of application (e.g. pre-filled syringes for s.c. application or spray (aerosol) containers for nasal and pulmonary use).

Thus, the advantages of the invention are:
- Maximization of benefit/risk ratio and/or
- Minimization of drug exposure and/or
- Improved patient convenience/acceptance and/or
- Reduction of infusion related side effects and/or
- Reduction of the rate of opportunistic infections and/or
- Reduction of thyroid abnormalities (incl. Graves' disease) and/or
- Reduction of autoimmune hematological complications (e.g. thrombocytopenia) and/or
- Reduction of other (serious) adverse events and/or
- Minimization of antibody formation to Campath-1 H

In certain embodiments of the invention two initial fixed treatment cycles which are separated by 1 - 24 months are followed by a third or subsequent treatment cycle(s) only upon evidence of renewed MS activity (i.e. "re-treatment as needed"). Thus the third and subsequent treatment cycle(s) may be initiated immediately prior to or subsequent to cessation of any symptomatic treatment (e.g. steroids) which may have been administered for the acute relapse (i.e. no fixed interval (time period) between subsequent treatment cycles). Alternatively, re-treatment upon renewed MS activity is only performed if at least 3 - 24 months have passed since the second (previous) treatment cycle.

In one embodiment of the invention, Campath-1 H is initially administered, or used for the production of a medicament to be initially administered (Month 0) at a dose of 10 mg/day for two consecutive days followed by a second fixed treatment cycle of 10 mg/day for two consecutive days at Month 12. Subsequent re-treatment is then only conducted on a re-treatment as needed basis with one or more additional cycles of 10 mg/day for two days.

In another embodiment of the invention, Campath-1H is initially administered, or used for the production of a medicament to be initially administered (Month 0) at a dose of 12 mg/day for five consecutive days followed by a second fixed treatment cycle of 12 mg/day for three consecutive days at Month 12. Subsequent re-treatment, is then only conducted on a re-treatment as needed basis with one or more additional cycles of 12 mg/day for three days.

It is anticipated that these treatment and use regimens will result in a sustained depletion of lymphocytes and a commensurate degree of clinical benefit while affording safety advantages over the regimens previously used in this patient population.

Without being bound by theory, these treatment and use regimens have been developed based in part on an analysis of Campath-1H pharmacokinetics and pharmacodynamics in patients enrolled in the CAMMS223 clinical trial. A variety of pharmacokinetic and pharmacodynamic models were developed for the purpose of this analysis. Model selection was based on physiological and pharmacological rationale and the principle of parsimony- simpler models were chosen over more complex models when statistically justified. First, exploratory data analysis was undertaken to examine the basic structure of the concentration-time data and to identify any outliers. Second, various structural models, such as the 2-compartment model, were developed without covariates. Once the basic structural model was identified, covariates were included in the model to see whether their inclusion improved the goodness of fit. Once the final pharmacokinetic model was identified, these parameters were fixed and the pharmacodynamic model was developed. Only one model type was examined, an indirect response model, which has been shown to be a good model for biomarkers such as hematologic indices. Once the final pharmacokinetic-pharmacodynamic model was identified, the parameters were fixed and deterministic simulations were done to examine the effect of alternate dosing strategies on lymphocyte counts.

The following dosing regimens/ uses were examined:
1. 5 days at 24 mg followed by yearly re-treatment/ use of 3 days at 24 mg;
2. 5 days at 12 mg followed by yearly re-treatment/ use of 3 days at 12 mg;
3. 2 days at 10 mg followed by yearly re-treatment/ use of 2 days at 10 mg;
4. 2 days at 10 mg followed by yearly re-treatment/ use of 1 day at 10 mg;
5. 5 days at 4 mg followed by yearly re-treatment/ use of 3 days at 4 mg;
6. 1 day at 10 mg followed by yearly re-treatment/ use of 1 day at 6 mg;
7. 1 day at 5 mg followed by yearly re-treatment/ use of 1 days at 3 mg; and
8. 1 day at 1 mg followed by yearly re-treatment/ use of 1 day at if mg.
These regimens were coded as day x dose for simplicity. So, for example, 5 daily doses of 12 mg would be coded as 5 x 12 mg, a single dose of 10 mg would be coded as 1 x 10 mg, etc.

The pharmacokinetic and pharmacodynamic modeling showed that Campath-1 H is an extremely potent depleter of lymphocytes. A single 5 mg dose can decrease lymphocytes by ~50% with a nadir occurring about 10 weeks after the dose. Further, the modeling showed that increasing dose resulted in greater lymphocyte depletion, with almost complete lymphocyte depletion seen with the 5 × 12 mg treatment group. One specific result of this analysis is the recognition that Campath-1 H treatment delivered in a cycle of 10mg/day for two days with re-treatment at 12 months with 10 mg/day for 2 days (i.e., the 20/20 mg regimen) is predicted to lead to a sustained lymphocyte depletion that is only modestly less than with higher doses. Given that the mechanism of action of Campath-1 H is presumed to be due to immune suppression, it is anticipated that a modest reduction in lymphopenia will only be associated with a comparably modest reduction in efficacy. Thus, the 20/20 mg regimen is expected to result in a moderately lesser degree of lymphocyte depletion compared with the regimens previously studied.

A lower Campath-1 H dosage delivered with fewer infusions is expected to trigger fewer acute infusion reactions and limit the potential for adverse events associated with intravenous (IV) injections in general. In view of the known immune-suppressing effects of Campath-1H, reduction in the administered dose may also result in a lower risk of infectious complications. The relative risk for autoimmune complications is also predicted to be lower.

The following examples demonstrate the feasibility of the invention, without restricting the invention only to these examples.

### Example 1

An open, rater blinded, randomized, multicenter trial in treatment naïve patients with early active relapsing-remitting multiple sclerosis is performed. The first treatment cycle of Campath-1H consists of 5 daily doses of 12 mg at Month 0. The second (fixed) treatment cycle consists of 3 daily doses of 12 mg at Month 12. Subsequently, re-treatment of patients with 3 daily doses of 12 mg Campath-1 H only occurs once evidence of renewed MS activity is observed in the respective patient. Thus, the third and subsequent cycles are administered only if the patients have evidence of renewed MS activity, in this example defined as at least 1 documented clinical relapse or presentation of at least 3 new MRI lesions (total) compared to the MRI following the prior Campath-1H treatment (i.e. "re-treatment as needed" regimen). If these criteria are fulfilled, the patient may be immediately retreated.

If a Campath-1H patient has received steroids for symptomatic treatment of a relapse within 2-8 weeks prior to a scheduled/planned alemtuzumab infusion, the infusion and pre-medication with steroids may be deferred until 2-8 weeks after steroid dosing for treatment of the relapse. Steroid pretreatment is typically administered on the first three days of the Campath-1 H infusion to avoid/minimize infusion related side effects. Consecutive treatment cycles will follow the same "re-treatment as needed" rules as described above.

The patients may receive pre-treatment with 1 g i.v. methylprednisolone over 1 hour on the first 3 days of each treatment cycle in order to ameliorate any cytokine release syndrome.

### Example 2

MS patients are treated as set forth above, except that the first treatment cycle of Campath-1H consists of 2 daily doses of 10 mg at Month 0 and the second treatment cycle consists of two daily doses of 10 mg at Month 12. Subsequently re-treatment of patients with 2 daily doses of 10 mg Campath-1 H only occurs once evidence of renewed MS activity is observed as described in Example 1 ("re-treatment as needed").

### Example 3

MS patients are treated using the same treatment regimens as outlined in Examples 1 or 2, except that the second and subsequent re-treatments are on a re-treatment as needed basis provided that at least 6 months have passed since the prior Campath-1 H treatment cycle (dosing period).

### Example 4,

MS patients are treated using the same treatment regimen as outlined in Example 3, however the patients are re-treated at any time after the initial (prior) Campath-1 H treatment cycle (dosing period) if evidence of renewed MS activity has been observed and if the respective patient has not received steroids for symptomatic treatment of a relapse within 2-8 weeks prior to the planned Campath-1 H cycle. If a patient has received steroids within 2-8 weeks prior to a planned Campath-1 H cycle, then re-treatment is started 2-8 weeks after completion of the steroid treatment.

### Example 5

MS patients are treated with two treatment cycles of Campath-1 H using dosing regimens as outlined in Examples 1 or 2. However, subsequent to the second cycle at Month 12, re-treatment occurs in 18 months intervals irrespective of renewed disease activity (fixed re-treatment).

All publications, including patents, cited in this disclosure are incorporated by reference in their entirety.

### Preferred embodiments of the present invention are described below and are referred to as embodiments E1 to E29.

E1. A method for the treatment of multiple sclerosis (MS) in a patient, comprising administration of a first cycle of Campath-1 H followed by at least one further cycle of Campath-1H , in which each treatment cycle comprises 1-5 doses which are applied on consecutive days, wherein the daily dose is >0 and ≤ 12 mg, and wherein each treatment cycle is separated from the next cycle by at least 1 - 24 months.
E2. The method of E1, wherein the at least one further cycle is administered at 12 months after the first cycle.
E3. The method of E1, wherein the at least one further cycle is administered upon detection of renewed MS activity.
E4. The method of E1, wherein said first cycle of Campath-1H is administered at a dose of 10 mg/day for two days.
E5. The method of E1, wherein said first cycle of Campath-1 H is administered at a dose of 12 mg/day for five days.
E6. The method of any one of E1-5, wherein the at least one further cycle of Campath-1 H is of the same duration and daily dose as the first cycle.
E7. The method of any one of E1-5, wherein the at least one further cycle of Campath-1 H is at the same daily dose for a shorter duration than the first cycle.
E8. The method of any one of E1-5, wherein the at least one further cycle of Campath-1 H is at a lower daily dose than the first cycle,
E9. The method of any one of E1-8, wherein the patient has relapsing MS.
E10. The method of E4, wherein said patient is retreated upon detection of renewed MS activity with a further cycle of Campath-1H at a dose of 10 mg/day for two days.
E11. The method of E4, wherein said patient is retreated at 12 months after said first cycle with a further cycle of Campath-1H at a dose of 10 mg/day for two days.
E12. The method of E5, wherein said patient is retreated at 12 months after said first cycle with a further cycle of Campath-1H at a dose of 92 mg/day for three days.
E13. The method of E5, wherein said patient is retreated upon detection of renewed MS activity with a further cycle of Campath-1H at a dose of 12 mg/day for three days.
E14. A method according to E1 or 2, wherein each treatment cycle is separated from the next cycle by at least 6 months.
E15. A method according to E1 or 2, wherein each treatment cycle is separated from the next cycle by at least 12 months.
E16. A method according to E1 or 2, wherein each treatment cycle is separated from the next cycle by at least 18 months.
E17. A method according to E1 or 2, wherein each treatment cycle is separated from the next cycle by at least 24 months.
E18. A method for the treatment of multiple sclerosis in a patient, comprising administration of Campath-1H at a dose of less than 12 mg/day for a period of 1-5 consecutive days.
E19. Use of Campath-1H for the production of a medicament for the treatment of multiple sclerosis, which medicament is administered at a dose of less than 12 mg/day for a period of 1-5 consecutive days.
E20. Use of Campath-1H for the production of a medicament for the treatment of multiple sclerosis (MS), comprising a first treatment cycle of Campath-1H followed by at least one further treatment cycle, in which each treatment cycle comprises 1-5 daily doses which are applied on consecutive days, wherein the daily dose is >0 and ≤ 12 mg, and wherein each treatment cycle is separated from the next cycle by at least 1 - 24 months.
E21. Use according to E19 or 20, wherein the daily dose is between 4 - 10 mg.
E22. Use according to E20, wherein each treatment cycle is separated from the next cycle by at least 6 months.
E23. Use according to E20, wherein each treatment cycle is separated from the next cycle by at least 12 months.
E24. Use according to E20, wherein each treatment cycle is separated from the next cycle by at least 18 months.
E25. Use according to E20, wherein each treatment cycle is separated from the next cycle by at least 24 months.
E26. Use according to E20, wherein the number of consecutive days of treatment per cycle is 1-3 days.
E27. Use according to any one of E20-26, wherein said at least one further treatment cycle is administered upon evidence of renewed MS activity.
E28. Use of Campath-1H for the production of a medicament for the treatment of multiple sclerosis (MS), comprising two initial fixed treatment cycles which are separated by 1-24 months are followed by a third or subsequent treatment cycle only upon evidence of renewed MS activity.
E29. Use of Campath-1H according to E28, wherein the third or subsequent treatment cycle is only performed if at least 3-24 months have passed since the second treatment cycle.

## Claims

1. Use of Campath-1H for the production of a medicament for the treatment of multiple sclerosis (MS) in a patient, the treatment comprising the cyclic application of Campath-1H in daily doses of up to 12 mg/day over a period of 1-5 days with each treatment cycle of Campath-1H being separated from the prior treatment cycle of Campath-1H by at least one month.

2. The use of claim 1, wherein the number of treatment cycles is limited to 2 - 10, preferably 2 - 5.

3. Use of Campath-1H for the production of a medicament for the treatment of multiple sclerosis (MS) in a patient, the treatment comprising the cyclic application of Campath-1H in daily doses of up to 12 mg/day over a period of 1-5 days, wherein re-treatment only occurs once evidence of renewed MS activity in the patient is observed.

4. Use of Campath-1H for the production of a medicament for the treatment of multiple sclerosis (MS) in a patient, the treatment comprising a first treatment cycle of Campath-1H followed by at least one further treatment cycle of Campath-1H, in which each treatment cycle comprises 1-5 doses which are applied on consecutive days, wherein the daily dose is >0 and ≤ 12 mg, and wherein each treatment cycle is separated from the next treatment cycle by at least 1 - 24 months.

5. The use of claim 4, wherein the at least one further treatment cycle of Campath-1H is at the same daily dose for a shorter duration than the first treatment cycle.

6. The use of claim 4 or claim 5, wherein the first treatment cycle of Campath-1H is at a dose of 12 mg/day for five days.

7. The use of claim 6, wherein the patient is to be retreated at 12 months after said first treatment cycle with a further treatment cycle of Campath-1H at a dose of 12 mg/day for three days.

8. The use of any one of claims 4 to 7, wherein two initial treatment cycles of Campath-1H are to be followed by a third or subsequent treatment cycle of Campath-1H only upon evidence of renewed MS activity.

9. The use of claim 8, wherein the third or subsequent treatment cycle is at a dose of 12 mg/day for three days.

10. Use of Campath-1H for the production of a medicament for the treatment of multiple sclerosis (MS) in a patient, the treatment comprising the administration of Campath-1H at a dose of less than 12 mg/day for a period of 1-5 consecutive days.

11. The use according to claim 10, wherein the treatment comprises an original treatment regimen comprising the administration of Campath-1H at a dose of less than 12 mg/day for a period of 1-5 consecutive days, and then retreating the patient using a second treatment regimen in which the dose and/or the duration is equal to or less than that of the original treatment regimen.

12. The use according to any preceding claim, wherein the patient has relapsing MS.

13. The use according to any preceding claim, wherein Campath-1H is to be administered intravenously.

14. The use according to any preceding claim, wherein methylprednisolone is applied before, during or after infusion of Campath-1H.

15. The use according to any preceding claim, wherein the patient has received prior therapy for MS.
